# EUROPÄISCHE PATENTANMELDUNG

(11) **EP 2 602 246 A1**
(43) Veröffentlichungstag der Anmeldung: **12.06.2013**
(21) Anmeldenummer: 11192380.1
(22) Anmeldetag: 07.12.2011
(51) Int. Cl.: C07D 207/267

(54) **Verfahren zur Reinigung von N-alkyl-substituierten Pyrrolidonen**

(71) Anmelder: BASF SE, 67056 Ludwigshafen (DE)
(72) Erfinder: Vogler, Thomas, Dr. rer nat, 67071 Ludwigshafen (DE); Pinkos, Rolf, Dr., 67098 Bad Dürkheim (DE); Ott, Karl, 68723 Plankstadt (DE)

(57) **Zusammenfassung**

Die Erfindung betrifft ein Verfahren zur Reinigung von N-Alkyl-substituierten Pyrrolidonen, die ungesättigte Verunreinigungen aufweisen, durch Behandeln derselben mit sauer wirkenden Verbindungen und anschließender Destillation

## Beschreibung

Die Erfindung betrifft ein Verfahren zur Reinigung von N-Alkyl-substituierten Pyrrolidonen, die ungesättigte Verunreinigungen aufweisen, durch Behandeln derselben mit sauer wirkenden Verbindungen und anschließender Destillation.

N-Alkyl-substituierte Pyrrolidone werden technisch durch die Umsetzung von Alkylaminen mit gamma-Butyrolacton hergestellt. So erhält man beispielsweise durch Umsetzung von Methylamin mit gamma-Butyrolacton N-Methylpyrrolidon (NMP), das technisch vielfältig als Lösungsmittel eingesetzt wird, wie von K. Weissermel, H.-J. Arpe in Industrielle Organische Chemie, 5. Auflage, Wiley-VCH, Seite 115 beschrieben. Die Reingewinnung des N M P erfolgt durch destillative Abtrennung von ggf. überschüssigem Methylamin sowie Reaktionswasser. Die erzielbaren Reinheiten sind sehr hoch und liegen deutlich über 99,5 %, gewöhnlich über 99,8 %. Andere Alkylamine, wie z.B. N-Ethylpyrrolidon, werden analog hergestellt.

Beim Einsatz von N-Alkylpyrrolidonen, z.B. als Lösemittel, werden diese in ihrer Zusammensetzung geändert, was einer direkten Wiederverwendung entgegensteht, obwohl diese, aus umwelttechnischen und ökonomischen Gründen, sehr wünschenswert wäre.

Eine Anwendung für N-Alkyl-substituierte Pyrrolidone und insbesondere NMP ist die Herstellung von Lithiumionen-Batterien als Lösungsmittel für die Beschichtungen der Anoden- und Kathodenträger. Hierfür sind besondere Anforderungen an das Lösemittel gestellt, insbesondere sollte die eingesetzte Qualität nicht signifikant schwanken und keine neuen, zuvor nicht auf ihren Prozesseinfluss getestete Nebenkomponenten, enthalten. So sollen die Metallionengehalte unter 5 ppb, Wassergehalte unter 300 ppm , die Gesamtreinheit (inklusive von 1,3- und 1,4-Dimethylpyrrolidion) über 99,8 % (sogenannte "electronic grade quality") liegen. Nach der ersten Verwendung von N-Alkyl-substituierten Pyrrolidonen kann es, neben der Erhöhung des Wassergehalts, zur Verunreinigung z.B. durch chemische Reaktion kommen, was eine wünschenswerte direkte Wiederverwendung des N-Alkyl-substituierten Pyrrolidons und insbesondere die Verwendung in der Herstellung von Lithiumionen-Batterien verhindert.

In WO 2011/030728 A1 wird eine Destillation von NMP beschrieben. Wie das Vergleichsbeispiel 1 dieser Anmeldung zeigt, in dem NMP, Verunreinigungen enthält, die einen höheren Oxidationsgrad als NMP selbst aufweisen, kann eine Destillation wie sie in WO 2011/030728 A1 beschrieben ist, nicht dazu eingesetzt werden, um die nötigen Reinheitsgrade des ursprünglich eingesetzten NMPs wieder zu erreichen. Reinheitsgrade wie das frisch eingesetzte N-Alkylpyrrolidon sind daher durch reine Destillation nicht zu erreichen und somit für das Recyceln von N-Alkyl-substituierten Pyrrolidonen, die in der Herstellung von Lithiumionen-Batterien eingesetzt werden, ungeeignet.

Für die Reinigung von N M P schlägt die Firma AMCEC auf der Internetseite http://www.amcec.com/nmp%20recovery.html ("AMCEC NMP Recovery System") vor, dass Verunreinigungen, per Filtration über Aktivkohle oder Zeolithe entfernt werden können. Gemäß dem in dieser Anmeldung durchgeführten Vergleichsbeispiel 2 sind jedoch N-Alkylpyrrolidone wie NMP, das Verunreinigungen mit höherem Oxidationsgrad als NMP enthält, nicht auf diese Art zu reinigen. Daher ist auch diese Reinigung per Filtration nicht geeignet für den Einsatz des N-Alkyl-substituierten Pyrrolidons als Lösungsmittel in der Batterieherstellung mit electronic grade quality.

Die Aufgabe der vorliegenden Erfindung ist es daher ein Verfahren bereit zu stellen, dass es ermöglicht, auf einfachem Wege, effizient und kostengünstig mit jedoch hohen Ausbeuten, N-Alkyl-substituierte Pyrrolidone in hohen Reinheitsgraden, die dem electronic grade entsprechen, wiederzugewinnen.

Die Aufgabe wird gelöst durch ein Verfahren zur Reinigung von N-Alkyl-substituierten Pyrrolidonen, die eine oder beide der Verunreinigungen der Formel I und II enthalten, 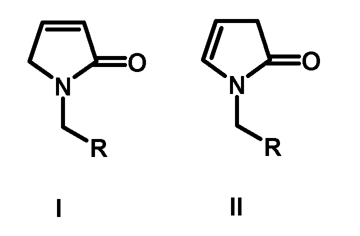 mit R ausgewählt aus der Gruppe von Wasserstoff, linearen oder verzweigten C₁-C₂₀-Alkylgruppen,
umfassend folgende Schritte
I) Bereitstellung einer Mischung enthaltend mindestens ein N-Alkyl-substituiertes Pyrrolidon, mindestens eine Verbindung der Formel I und II in Mengen von 1 bis 10000 ppm
II) Umsetzung der Mischung aus Schritt I) mit mindestens einer sauer wirkenden Substanz
III) Destillation der Mischung aus Schritt II)

In den Verunreinigungen der Formel I und II steht der Rest R für Wasserstoff, lineare oder verzweigte C₁-C₂₀-Alkylreste. Bevorzugt ist R Wasserstoff oder ein linearer oder verzweigter C₁ - C₁₀ Alkylrest. Besonders bevorzugt ist R ausgewählt aus der Gruppe von Wasserstoff, Methyl, Ethyl, n-Propyl und n-Butyl. Ganz besonders bevorzugt ist R ausgewählt aus der Gruppe von Wasserstoff und Methyl. Insbesondere ganz besonders bevorzugt ist R Wasserstoff.

Die Menge dieser Verunreinigungen der Formel I und II im N-Alkyl-substituierten Pyrrolidon liegt im Allgemeinen einzeln oder als Mischung beider im Bereich von 1 bis 10000 ppm, bevorzugt zwischen 1 und 5000 ppm, besonders bevorzugt zwischen 1 und 2500 ppm, bezogen auf die Menge der zu reinigenden Mischung. Im zu reinigenden N-Alkyl-substituierten Pyrrolidon können zu den Verunreinigungen der Formel I und II auch alkyl-substituierte Derivate oder weitere Verunreinigungen mit höherem Oxidationsgrad als NMP vorliegen. Diese Verunreinigungen können einzeln oder als Mischung einzelner oder aller vorliegen. In den Verunreinigungen steht der Rest R1 für lineare oder verzweigte C₁-C₂₀-Alkylreste. Bevorzugt ist R1 ein linearer oder verzweigter C₁ - C₁₀ Alkylrest. Besonders bevorzugt ist R1 ausgewählt aus der Gruppe Methyl oder Ethyl.

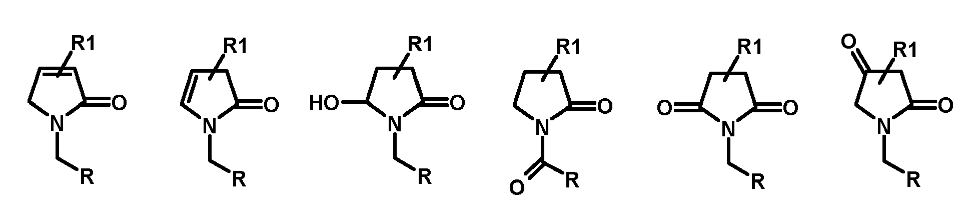

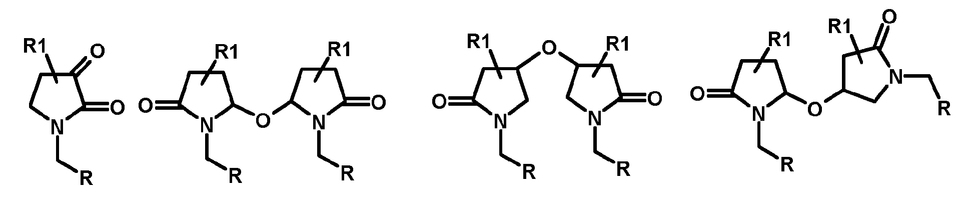

Der Wassergehalt des zu reinigenden N-Alkyl-substituierten Pyrrolidon liegt im Allgemeinen zwischen 0,05 % bis 90 %, bevorzugt zwischen 0,1 % bis 50 % und besonders bevorzugt zwischen 0,2 % - 10 %.

Die gemäß Schritt II des erfindungsgemäßen Verfahrens eingesetzte saure Substanz ist ausgewählt aus der Gruppe aus Bronsted und Lewis-sauren Verbindungen. Bevorzugt werden protische anorganische Säuren, Carbonsäuren, Sulfonsäuren und Lewis-saure Metalloxide. Besonders bevorzugt sind die sauren Substanzen ausgewählt aus der Gruppe von Phosphorsäure, Schwefelsäure, kurz- und langkettigen Carbonsäuren, Ionentauscher, Heteropolywolframsäuren und aktivierte Schichtsilikate. Ganz besonders bevorzugt ist die saure Substanz ausgewählt aus der Gruppe von Phosphorsäure, Schwefelsäure und sulfonierten Ionentauscher.

Die sauren Verbindungen können in homogen löslicher Form oder als Heterogenverbindungen eingesetzt werden. Wird ein homogen löslicher Katalysator eingesetzt so ist dieser bevorzugt Bronsted sauer. Besonders bevorzugt ist der Einsatz von Verbindungen, die in Schritt III des erfindungsgemäßen Verfahrens leicht vom zu reinigenden Pyrrolidon abgetrennt werden können.

Die sauren Verbindungen werden beispielsweise in Mengen zwischen 5 und 50000 ppm, bevorzugt zwischen 25 bis 25000 ppm, besonders bevorzugt zwischen 50 und 10000 ppm bezogen auf die Menge der zu reinigenden Mischung zugesetzt. Die Menge an sauren Verbindungen richtet sich nach der Menge an Verunreinigung, kann dabei sowohl katalytisch als auch stöchiometrisch eingesetzt werden. Bevorzugt ist die überstöchiometrische Zugabe der sauren Verbindung zur Menge an Verunreinigung.

Die sauren Verbindungen können in Substanz oder in Lösung oder als Suspension eingebracht werden. Als Lösemittel sind bevorzugt Wasser, das herzustellende N-Alkyl-substituierte Pyrrolidon, Ether wie z.B. Tetrahydrofuran und Diethylether. Besonders bevorzugt ist das herzustellende N-Alkyl-substituierte Pyrrolidon.

Die Zugabe der sauren Substanz, die zum verunreinigten N-Alkyl-substituierten Pyrrolidon, insbesondere zum verunreinigten N M P, hinzugefügt wird, kann bei Temperaturen zwischen 10 und 350°C erfolgen. Bevorzugt ist das erfindungsgemäße Verfahren, wenn die Temperatur und Verweilzeit der sauren Substanz auf einander abgestimmt ist. Generell gilt, je höher die Temperatur, desto geringer ist die notwendige Verweilzeit, die benötigt wird, um den gewünschten Reinigungseffekt zu erhalten. So liegen die Verweilzeiten beispielsweise von 0,1 bis 10 Stunden bei Temperaturen zwischen 50 und 250 °C, wobei während des erfindungsgemäßen Verfahrens die Temperatur nicht konstant gehalten werden muss. Die notwendige Verweilzeit für den Prozess setzt sich zusammen aus der Verweilzeit, in der das verunreinigte N-Alkyl-substituierte Pyrrolidon zusammen mit der sauren Substanz umgesetzt wird und der Verweilzeit, die während der Destillation eingestellt wird. Ist die Verweilzeit während der Destillation bereits ausreichend, so kann ein separater Verweilzeitschritt entfallen.

Die Destillation kann diskontinuierlich oder kontinuierlich erfolgen. Dabei können für den Fachmann alle bekannten Destillationskolonnen eingesetzt werden.

Beispielsweise kann man, beim Einsatz eines absatzweisen Prozesses, wie folgt vorgehen: Man legt in einem ersten Schritt das verunreinigte N-Alkyl-substituierte Pyrrolidon, insbesondere NMP, zusammen mit der sauren Substanz in einem Rührkessel, bevorzugt in einer Inertgasatmosphäre, vor, vermischt die Stoffe z.B. durch Rühren oder Umpumpen und heizt, ggf. unter Druck, auf die gewünschte Temperatur auf und lässt das Gemisch reagieren. Anschließend pumpt man im zweiten Schritt den Inhalt in eine oder mehrere Kolonnen und trennt (absatzweise oder kontinuierlich) Leichtsieder wie z.B. Wasser und Hochsieder, die ggf. die im Überschuss zugegebene saure Substanz und gebildete Hochsieder enthalten, vom N-Alkylsubstituiertem Pyrrolidon ab.

Der zuvor beschriebene erste Schritt, kann auch in einer Kolonne ablaufen, bevor destilliert wird. Der zuvor beschriebene erste Schritt kann ebenso kontinuierlich durchgeführt werden. Dabei kann wiederum ein der Destillation vorgeschalteter separater Behälter oder eine Destillationskolonne verwendet werden.

Eine bevorzugte Ausführungsform des Verfahrens ist, das verunreinigte N-Alkyl-substituierte Pyrrolidon und insbesondere das verunreinigte NMP, zu einem Reaktionsaustrag hinzuzugeben der durch Umsetzung von gamma-Butyrolacton mit dem entsprechendem Amin zum N-Alkyl-substituierten Pyrrolidon, wie Methylamin zu NMP, entstanden ist. Dieses Gemisch enthält im Allgemeinen N-Alkyl-substituiertes Pyrrolidon, Wasser und das entsprechende Amin. Bevorzugt wird bei der Zugabe der sauren Verbindung zu diesem Reaktionsaustrag auch die Menge an Amin berücksichtigt und die saure Verbindung überstöchiometrisch zur Menge an Verunreinigung und Amin zugegeben.

Dieses Gemisch enthält im Allgemeinen N-Alkyl-substituiertes Pyrrolidon, Wasser und das entsprechende Amin. Diese Verfahrensvariante wird bevorzugt kontinuierlich ausgeübt. Dabei wird in einer ersten Kolonne ein Gemisch von Amin, insbesondere Methylamin für die Reinigung von NMP, und Wasser über Kopf abgetrennt (Kolonnendruck 300 - 5000 mbar, Sumpftemperaturen 100 -250 °C für die Reinigung von verunreinigtem NMP), das Sumpfprodukt gelangt in eine zweite Kolonne (Kolonnendruck 20 - 500 mbar, Sumpftemperaturen 120 bis 230 °C, für die Reinigung von verunreinigtem NMP), bei der Reste an Wasser und Amin, insbesondere Methylamin für die Reinigung von NMP, über Kopf abgetrennt werden und das Sumpfprodukt in eine dritte Kolonne (Kolonnendruck 20 - 300 mbar, Sumpftemperaturen 120 bis 230 °C, für die Reinigung von verunreinigtem NMP) gelangt, bei dem reines N-Alkyl-substituiertes Pyrrolidon, insbesondere NMP, über Kopf oder über einen Seitenabzug abgetrennt wird, während über Sumpf Hochsieder ausgeschleust werden. Es gibt bei dieser Destillationssequenz auch Varianten, wie z.B. dass in der zweiten Kolonne über Kopf Wasser und Amin, insbesondere Methylamin für die Reinigung von NMP, ausgeschleust werden, über Seitenabzug N-Alkyl-substituiertes Pyrrolidon, insbesondere NMP, und über Sumpf Hochsieder. Das N-Alkyl-substituierte Pyrrolidon, insbesondere NMP, kann je nach Reinheitsanforderungen dann in einer dritten Kolonne, wie beschrieben, weiter gereinigt werden. Ist die zweite Kolonne als Trennwandkolonne ausgeführt, so kann das N-Alkyl-substituierte Pyrrolidon, insbesondere NMP, als Seitenabzug bereits sehr rein gewonnen werden und eine dritte Kolonne kann entfallen.

Die verwendeten Apparate sind aus gängigen Edelstählen gefertigt, ebenso alle Rohrleitung und Kolonneneinbauten wie Packungen usw. Um N-Alkyl-substituierte Pyrrolidone, insbesondere NMP in "Electronic-grade"-Qualität zu erhalten, empfiehlt es sich, auch die verwendeten Tanks in Edelstahl auszuführen und mit Schutzgas wie z.B. Stickstoff zu überlagern. Ggf. wird das N-Alkyl-substituierte Pyrrolidon, insbesondere NMP, zur Entfernung von Metallionen und/oder freien Aminspuren zusätzlich noch über Ionentauscher geleitet.

### Beispiele

Zur Verdeutlichung der Erfindung sollen die nachfolgenden Beispiele dienen. Die in den Beispielen angegebenen Gehalte an Verunreinigungen sind durch Gaschromatographie (GC-Gerät HP6890, FID-Dektor, Stickstoff Trägergas mit 1,0 mL/min (const. flow); Split Ratio 1:50; Säule RTX-1, 30 m, 0,32 mm, 1,0 µm Film; Temperaturprogramm Start bei 80 °C, dann 5 °C/min bis 140 °C, dann 5 °C/min bis 200 °C und 10 min isotherm, dann 10 °C/min bis 340 °C und 8 min isotherm) bestimmt. Alle Versuche wurden unter einer Stickstoffatmosphäre durchgeführt. Wasserwerte wurden per Karl-Fischer-Titration bestimmt. Eingesetzt wurden N M P sowie die entsprechenden Verunreinigungen wie in den Beispielen angegeben.

### Vergleichsbeispiel 1

### Destillation, wie sie in WO-A1 2011/030728 beschrieben wird.

Ein verunreinigtes NMP, mit 3 % Wasser und 300 ppm Verunreinigungen ausgewählt aus Verunreinigungen der Formeln I und II (R = H, Dehydro-NMP[TV1]), wurde fraktioniert in einer 110 cm langen Füllkörperkolonne (Sulzer-Pack) mit Rücklaufteiler bei Sumpftemperaturen zwischen 130 bis 140°C und Drücken zwischen zu Beginn 1000 mbar (Wasserabtrennung) und 90 mbar Kopfdruck bei einem Rücklauf- zu Abnahme-Verhältnis von 50 zu 1 destilliert. Es wurden ausgehend von 2 kg Vorlage, insgesamt 16 Destillatfraktionen genommen. In den ersten beiden Fraktionen, die bei Kopftemperaturen von 55 - 118 °C erhalten wurden, fand sich überwiegend Wasser (> 98 %). Bei 100 mbar und einer Kopftemperatur von 122 - 126°C wurde eine Fraktion mit 0,7 % Wasser und N M P mit über 99 % erhalten. Die nachfolgenden Fraktionen 4 bis 13 (zusammen ca. 1,5 kg) enthielten Wasser unter 1000 ppm, N M P in Reinheiten über 99,8 % allerdings noch die bereits in der Vorlage vorhandenen Verunreinigungen. Ab Fraktion 14 (zusammen ca. 300 g) war das NMP frei von den unerwünschten Verunreinigungen. Der Versuch zeigt, dass alleine durch Destillation, nur ein unwirtschaftlich kleiner Teil an NMP (ca. 15 %) als NMP mit gewünschter Reinheit wiedergewonnen werden kann.

### Vergleichsbeispiel 2

Das Edukt wie in Vergleichsbeispiel 1 wurde über 500 mL Aktivkohle filtriert und anschließend wie in Vergleichsbeispiel 1 destilliert. Bereits nach der Filtration zeigte die GC-Analyse, dass kein Abreicherungseffekt der organischen Verunreinigungen eingetreten war und auch das Destillationsergebnis war analog Vergleichsbeispiel 1.

### Vergleichsbeispiel 3

Vergleichsbeispiel 2 wurde wiederholt, anstelle von Aktivkohle wurde ein Zeolith verwendet. Es ergab sich das gleiche Ergebnis wie in Beispiel 2.

### Erfindungsgemäße Beispiele

### Beispiel 1

Das Einsatzgemisch aus Vergleichsbeispiel 1 wurde mit 5 Gew.-% Amberlite IR120 H versetzt und 1 h auf 80 °C erhitzt. Lt. gaschromatographischer Analytik, waren die unerwünschten Verunreinigungen nicht mehr nachweisbar. Eine anschließende Destillation wie in Vergleichsbeispiel 1 ergaben ca. 90 % NMP in der gewünschten Reinheit, wobei der überwiegende Rest nur durch zu hohe Wassergehalte (> 1000 ppm) verunreinigt war.

### Beispiel 2

Das Einsatzgemisch aus Vergleichsbeispiel 1 wurde mit 5 Gew.-% Amberlite IR120 H versetzt und direkt bei 100 mbar Kopfdruck destilliert. Nach Wasserabrennung lag die Sumpftemperatur zwischen 125 und 135 °C. Lt. gaschromatographischer Analytik, waren die unerwünschten Verunreinigungen nicht mehr nachweisbar. Es wurden ca. 90 % NMP in der gewünschten Reinheit erhalten, wobei der überwiegende Rest nur durch zu hohe Wassergehalte (> 1000 ppm) verunreinigt war.

### Beispiel 3

Das Einsatzgemisch aus Vergleichsbeispiel 1 wurde mit 5 Gew.-% Nafion H versetzt und 1 h auf 110 °C erhitzt. Lt. gaschromatographischer Analytik, waren die unerwünschten Verunreinigungen nicht mehr nachweisbar. Eine anschließende Destillation wie in Vergleichsbeispiel 1 ergaben ca. 95 % NMP in der gewünschten Reinheit, wobei der überwiegende Rest nur durch zu hohe Wassergehalte (> 1000 ppm) verunreinigt war.

### Beispiel 4

Das Einsatzgemisch aus Vergleichsbeispiel 1 wurde mit 1000 ppm Phosphorsäure versetzt und 2 h auf 105 °C erhitzt. Lt. gaschromatographischer Analytik, waren die unerwünschten Verunreinigungen nicht mehr nachweisbar. Eine anschließende Destillation wie in Vergleichsbeispiel 1 ergaben ca. 95 % N M P in der gewünschten Reinheit, wobei der überwiegende Rest nur durch zu hohe Wassergehalte (> 1000 ppm) verunreinigt war.

### Beispiel 5

Das Einsatzgemisch aus Vergleichsbeispiel 1 wurde mit 2000 ppm Schwefelsäure versetzt und 2 h auf 115 °C erhitzt. Lt. gaschromatographischer Analytik, waren die unerwünschten Verunreinigungen nicht mehr nachweisbar. Eine anschließende Destillation wie in Vergleichsbeispiel 1 ergaben ca. 95 % N M P in der gewünschten Reinheit, wobei der überwiegende Rest nur durch zu hohe Wassergehalte (> 1000 ppm) verunreinigt war.

### Beispiel 6

Analog Beispiel 2 wurde N-Ethylpyrrolidon, das mit 300 ppm Verunreinigungen ausgewählt aus Verunreinigungen der Formeln I und II (R = Me, Dehydro-NEP) verunreinigt war, eingesetzt. Die Zusatzmenge an Amberlite IR120 H betrug 5 Gew.-%. Es wurden über 95 % reines N-Ethylpyrrolidon erhalten, das kein Succinimid mehr enthielt.

## Patentansprüche

1. Verfahren zur Reinigung von N-Alkyl-substituierten Pyrrolidonen, die eine oder mehrere der Verunreinigungen der Formel I bis II enthalten, 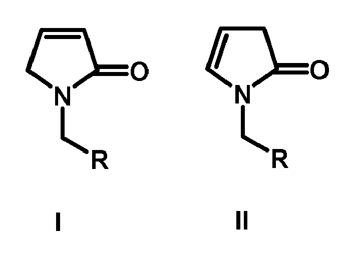 mit R ausgewählt aus der Gruppe von Wasserstoff, linearen oder verzweigten C₁-C₂₀-Alkylgruppen,
umfassend folgende Schritte
I) Bereitstellung einer Mischung enthaltend mindestens ein N-Alkyl-substituiertes Pyrrolidon, mindestens eine Verbindung der Formel I und II in Mengen von 1 bis 10000 ppm
II) Umsetzung der Mischung aus Schritt I) mit mindestens einer sauer wirkenden Substanz
III) Destillation der Mischung aus Schritt II)

2. Verfahren nach Anspruch 1, wobei die sauer wirkenden Substanzen ausgewählt sind aus der Gruppe von protischen anorganische Säuren, Carbonsäuren, Sulfonsäuren und Lewis-sauren Metalloxiden.

3. Verfahren nach einem der Ansprüche 1 bis 2, wobei die sauer wirkenden Substanzen sulfonierte Ionentauscher und/oder Phosphorsäure und/oder Schwefelsäure sind.

4. Verfahren nach einem der Ansprüche 1 bis 3, wobei die sauer wirkende Substanz in Mengen von 5 bis 50000 ppm bezogen auf die Menge an zu reinigender Mischung aus Schritt I) des erfindungsgemäßen Verfahrens zugesetzt wird.

5. Verfahren nach einem der Ansprüche 1 bis 4, wobei während einer Zeit von 0,1 bis 10 Stunden die Verunreinigung der Formel I und II während des Schritt II und/oder des Schritt III mit der sauer wirkenden Substanz bei gegebenenfalls wechselnden Temperaturen im Bereich von 50 bis 250°C in Kontakt stehen.

6. Verfahren nach einem der Ansprüche 1 bis 5, wobei die Zugabe der sauren Substanz in Schritt II) kontinuierlich erfolgt.

7. Verfahren nach einem der Ansprüche 1 bis 6, wobei die Destillation in Schritt III) kontinuierlich durchgeführt wird.

8. Verfahren nach einem der Ansprüche 1 bis 7, wobei die Destillation in Schritt III) in wenigstens zwei Kolonnen erfolgt.

9. Verfahren nach einem der Ansprüche 1 bis 8, wobei das N-Alkyl-substituierte-Pyrrolidon N-Methylpyrrolidon ist.

10. Verfahren nach Anspruch 9, bei dem die Destillation in Schritt III) bei Temperaturen im Bereich von 100 bis 250°C und Drücken im Bereich von 300 bis 5000 mbar durchgeführt wird.
